# EUROPEAN PATENT APPLICATION

(11) **EP 1 167 522 A1**
(43) Date of publication of application: **02.01.2002**
(21) Application number: 00908008.6
(22) Date of filing: 10.03.2000
(51) Int. Cl.: C12N 15/09, C12Q 1/00, C07K 4/00, C12Q 1/02, C12Q 1/70

(54) **METHOD FOR SCREENING BIOMOLECULE ACTIVITY REGULATOR**

(30) Priority: 10.03.1999 JP 6311099
(71) Applicant: Ajinomoto Co., Inc., Tokyo 104-0031 (JP)
(72) Inventor: OKAMOTO, Satoru Ajinomoto Co., Inc., Kawasaki-shi, Kanagawa 210-0801 (JP); MIWA, Kiyoshi Ajinomoto Co., Inc., Kawasaki-shi, Kanagawa 210-0801 (JP); ETO, Yuzuru Ajinomoto Co., Inc., Kawasaki-shi, Kanagawa 210-0801 (JP)
(74) Representative: HOFFMANN - EITLE
(86) International application number: JP0001478
(87) International publication number: WO0053740

(57) **Abstract**

A method for screening a low molecular weight substance which binds to a specific site of a biomolecule and may be usable as a starting material in drug development. This method comprises screening a substance that interacts with a specific region of a biomolecule having an activity, to regulate the activity, by the following steps:
(a) a step of selecting a recombinant organism that interacts with the biomolecule, from a peptide library composed of a collection of recombinant organisms each presenting at least one of various peptides on its surface; and
(b) a step of selecting a substance inhibiting the interaction between the selected recombinant organism or a peptide presented by the recombinant organism and the biomolecule.

## Description

### Technical Field

The present invention relates to a method for screening a substance regulating an activity of a biomolecule. In particular, the present invention provides a method useful for screening of a compound that may be used as, for example, a starting material of a drug.

### Background Art

Lead compounds, which are used as starting materials for development of drugs, have been screened by assaying, in a test tube, secondary metabolites produced by microorganisms or low molecular weight compounds chemically synthesized by referring to known molecular structures, or evaluating such substances by utilizing cells into which a reporter gene or the like is incorporated. Recently, a large scale random screening has been started by drug manufacturers by combining the high throughput screening, in which mechatronics technologies are applied to drug screening so that a system for the aforementioned assay is automatically operated in a short time, and the combinatorial chemistry technique, in which various compounds are automatically synthesized.

In recent years, a method has been known in which a collection of organisms expressing and presenting random amino acid sequences, that is, a peptide library, is constructed and sequences that can bind to a specific biomolecule are selected from this library. As such a library, the phage display random peptide library using a coat protein of filamentous phage has been reported (Scott, J.K. et al., Science, 249: 386-390, 1990). Libraries using such organisms and chemically synthesized libraries prepared on solid phase supports (Lam, K.S. et al., Nature, 354, 82, 1991) have been established as means for retrieving lead peptide compounds of drugs. For example, the aforementioned phage display random peptide library is used to retrieve lead peptide compounds useful in development of drugs such as hormone-mimic peptides (Wrighton, N.C. et al., Science, 273, 458, 1996 and Cwirla, S.E. et al., Science, 276, 1696, 1997) and receptor-ligand binding inhibitors (Martens, C.L. et al., J. Biol. Chem., 270, 21129, 1995).

However, while bioactivity of peptides retrieved from these peptide libraries can be improved by addition, substitution or the like of an amino acid so that an efficacy is exhibited even with a lower dosage, various problems mentioned below are expected when the peptides are used as therapeutic agents as they are. For example, problems often arise that bioavailability is low due to their fast metabolism in a living body, that administration conditions as a drug are limited due to their poor solubility, that peptides are easily recognized by the immune system due to their relatively high molecular weight and so forth. That is, it is readily expected that pharmacological actions of peptides as they are in humans would be low and thus it would be extremely difficult to develop drugs with such peptides. To overcome these problems, replacement of peptides with low molecular weight non-peptidic compounds that mimic three-dimensional structures of the peptides has long been attempted. However, this method is technically very difficult and there has been no report on examples in which practical drugs are successfully developed.

Meanwhile, as a method for identifying ligands that can bind to at least one determinant in a biologically active site on a target, a method using a reporter antibody selected from a divergent antibody library is known (Japanese Patent Unexamined Publication in Japanese (KOHYO) No. 10-507517). In this method, a target substance (ligand) is screened by using a variable region of an antibody selected from a phage library by detecting binding to a specific protein as an index. The binding affinity of an antigen protein and an antibody protein is considerably strong due to their large binding region. Therefore, when a library of compounds having a low molecular weight of several hundred daltons on average is screened for active substances by using the antibody-presenting phage as a probe and the degree of inhibition for binding to the target protein as an index, it is anticipated that competitive substances cannot be retrieved unless the substances can very strongly interact by themselves. Further, it is also readily expected that it is highly possible that substances that bind to regions unrelated to the protein function are selected since the antibody molecules recognize complicated structures of proteins as their characteristics.

### Disclosure of the Invention

The present invention was accomplished in view of the current situation described above, and an object thereof is to provide a method for screening a low molecular weight compound free from the problems described above, which is a substance that binds to a specific site of a biomolecule and thus may be used as a starting material in development of drugs.

To achieve the aforementioned object, the inventors of the present invention selected phage presenting relatively short random peptides for screening of a lead compound. That is, they considered that peptides having a molecular weight close to that of usual drugs (about several hundreds) would be more suitable to search for a further limited region (hot spot) within the functional region involved in an interaction between biomolecules. In other words, they considered that it would be advantageous to screen various biomolecule activity regulators having low molecular weights by using a peptide presented by phage as a probe because of its binding affinity tens to hundreds times lower (micromole (µM) order) than that of the antibody presented by phage. Further, it was also expected that a binding of low molecular weight peptide and a biomolecule that was irrelevant to the biomolecule function would be more unlikely to be selected compared with a case where an antibody molecule was used.

It has been shown that methods for selecting peptide compounds by utilizing an interaction between molecules as an index, of which typical example is the method utilizing a phage display random peptide library, are useful to retrieve peptide molecules inhibiting interactions between proteins or peptide molecules mimicking cytokine activities. Further, in general, interactions between proteins such as cleavage of a substrate protein by a protease, phosphorylation of a specific substrate molecule by a phosphorylating enzyme, and binding of a receptor present on a cell surface and a ligand are attained by characteristic amino acid residues exposed and localized on the surface of the proteins, specifically, amino acids having positively or negatively charged side chains or hydrophobic side chains. The present inventors found from the results of their various researches using phage display random peptide libraries that it was highly possible that peptides having an ability to bind to a specific protein selected from a phage display random peptide library would interact with an important functional region of the protein and that identification of such a peptide was equivalent to elucidation of the functional region of the protein.

Meanwhile, it is evident that serine protease, nonstructural protein 3 (hereafter, abbreviated as "NS3 protease") or cpro-2 encoded in the genome of human hepatitis C virus (hereafter, abbreviated as "HCV") plays an important role in proliferation of the virus (refer to Patick, A.K. et al., Clinical Microbiology Reviews, 11 (4), 614-627, 1998 and so forth). Since a drug inhibiting this NS3 protease activity is considered to be effective in prevention. or treatment of various liver diseases induced by infection and proliferation of HCV, for example, cirrhosis and liver cancer, such a drug is being searched for around the world, but no promising drug has been found yet. The present inventors obtained the aforementioned conception while searching for a drug inhibiting the NS3 protease. Then, they found a plurality of phage clones having affinity for the NS3 protease from phage random peptide libraries. They also found that these oligopeptides inhibited a substrate cleavage reaction by the NS3 protease. Moreover, they found a drug inhibiting the binding of the NS3 protease to phage presenting the oligopeptides, from chemical libraries by an ELISA system for detecting the binding and showed that this drug could inhibit the NS3 protease activity. The present invention was accomplished based on these findings.

That is, the present invention provides a method for screening a substance which interacts with a specific region of a biomolecule having an activity, to regulate the activity, said method comprising the following steps:
(a) a step of selecting a recombinant organism that interacts with the biomolecule from a peptide library composed of a collection of recombinant organisms each presenting at least one of various peptides on its surface with a proviso that the interaction is not an antigen-antibody reaction; and
(b) a step of selecting a substance inhibiting the interaction between the selected recombinant organism or a peptide presented by the recombinant organism and the biomolecule.

In a preferred embodiment of the present invention, the biomolecule is a protein, a nucleic acid or a sugar chain.

In a preferred embodiment of the present invention, the peptide library is composed of random peptide-presenting phage or random peptide-presenting *Escherichia coli*.

In a preferred embodiment of the present invention, the peptides each have a length of 3 to 15 residues.

In a preferred embodiment of the present invention, the selected recombinant organism or the peptide presented by the recombinant organism is labeled with a labeling substance.

A substance selected by the method of the present invention can be used as a low molecular weight lead compound of a drug because the substance can interacts with a specific region of a biomolecule such as a protein, a nucleic acid or a sugar chain to regulate its activity.

In the present invention, the "interaction" means that a molecule in a living body approaches another biomolecule to exhibit its function and induces a certain change in each of them. The expression of "regulating an activity of a biomolecule" includes reducing, eliminating or increasing the activity of the biomolecule, or reducing, eliminating or increasing activation or inactivation of the biomolecule.

The present inventors found from results of screening of peptides binding to a target protein using phage peptide libraries that most of the selected peptides bound to a region involved in the target protein function and few peptides were selected based on meaningless binding. For example, when a phage peptide library was screened by using IgG protein in order to select an epitope peptide of an antibody, peptide sequences that specifically bind to regions involved in other functions such as Fc regions of the IgG protein, i.e., an Fc receptor-binding site, and a complement protein-binding site, were selected in addition to the peptide sequence recognized and selected as the epitope.

Further, the maltose-binding protein (MBP) is widely utilized as a tag protein because it can be stably expressed in a large amount in *Escherichia coli* and purified by simple affinity purification. When binding peptides are selected by using a protein fused with this protein, peptide sequences specifically binding to MBP were also selected in addition to peptides specifically binding to the fused protein. That is, selection of peptide binding to a specific protein from a phage peptide library is exactly the same as searching of a peptide binding to the functional domain of the protein.

The following possibilities are implied from our results. If the protein is a receptor, a peptide binding to an extracellular ligand-binding site or an intracellular signal-transmitting site may be selected. If the protein is a ligand, a peptide binding to a receptor-binding site or a dimer-forming site may be selected. If the protein is an enzyme, a peptide binding to a substrate-recognizing site or an activator-binding site may be selected. If the protein is a transcription factor, a peptide binding to a DNA-binding site, a ligand-recognizing site or sites involved in interactions with other transcription factors may be collected.

Specific examples of the biomolecule to which the method of the present invention is applicable include NS3 protease of HCV, integrin, matrix metalloprotease, selectin and so forth.

As described above, a peptide that interacts with a biomolecule selected from a peptide library binds to a region involved in the function of the biomolecule. Therefore, it is highly possible that a substance inhibiting such an interaction between the peptide and the biomolecule also interacts with the biomolecule and binds to a region involved in the function. This strongly suggests that the function (activity) of the biomolecule can be regulated.

The present inventors designated a peptide binding to a specific region (functional region) of a protein as a surrogate peptide. That is, in the method provided by the present invention, as a first step, a phage presenting a peptide molecule that binds to a functional region of a protein, that is, a surrogate peptide, is retrieved by using a library composed of various molecules, for example, a phage random peptide library, and as a second step, a compound inhibiting the interaction between the protein and the surrogate phage is selected from various compound libraries. Then, as a third step, which is optionally performed, a biochemical activity of the selected compound, that is, enzyme inhibition activity or activity for inhibiting binding of the protein is biochemically evaluated by using an appropriate assay. By using the above-described method, a method for efficiently selecting compounds having an activity for regulating an interaction between proteins can be provided.

### Brief Description of the Drawings

Fig. 1 shows affinities for MBP-NS34a of 12 kinds of phage clones having peptides selected according to the present invention, which were determined by the ELISA method.
Fig. 2 shows effect of addition of the NS4a peptide on the binding of a phage having peptide selected according to the present invention to MBP-NS34a, which were determined by the phage ELISA method. Binding of the phage peptide K13 was inhibited the binding in a concentration-dependent manner.
Fig. 3 shows effect of the protease inhibitor HCP1271 on the binding of various phages to NS34a, which were determined by the phage ELISA method. Binding of clones other than the phage peptide K13 was inhibited in a concentration-dependent manner.
Fig. 4 shows effect of the protease inhibitor HCP1231 on the binding of various phages to NS34a, which were determined by the phage ELISA method. Binding of any clone was not inhibited.

### Best Mode for Carrying out the Invention

Examples of the peptides or organisms presenting the peptides used in the present invention include random peptide-presenting phage or random peptide-presenting *Escherichia coli*. To obtain the random peptide-presenting phage, for example, the phage random peptide library method described below can be used. The kind and the preparation method of the phage random peptide library are not particularly limited, and one prepared by the known method (Nishi T. et al., Jikken Igaku, 11, (3), 1759-1764) may be used. However, commercially available phage random peptide libraries, for example, Ph.D Phage Display System (New England Biolab Inc.) or the like may be purchased and used. As a specific method for constructing a phage random peptide library, for example, random synthetic genes coding for about 6 to 15 amino acids may be ligated to the gene for an N-terminus region of a coat protein of M13 phage (for example, gene III protein) and phage particles may be prepared by using phage DNA containing this gene. Examples of such a method include those reported by Scott, J.K. and Smith, G.P., Science, 249, 386, 1990; Cwirla, S.E. et al, Proc. Natl. Acad. Sci. USA, 87, 6378, 1990 and so forth. The phage DNA is not particularly limited so long as it can form a phage particle, but a phagemid vector is preferred.

When a phage random peptide library is prepared, it is preferred that the peptide presented by phage has a length of 3 to 15 residues. The peptide presented by phage may be directly presented by a surface layer protein of a phage particle or may be presented via a spacer having an appropriate length. Examples of the sequence of the spacer include an amino acid sequence known to successfully present a protein, such as GGGS (SEQ ID NO: 20), or a peptide sequence having functions as both of an epitope and a spacer, such as an antibody epitope sequence.

As the random peptide-presenting *Escherichia coli* library, a library in which random peptides are presented on the surface layer of flagellin proteins, which constitute a flagellum of *Escherichia coli*, is commercially available from Invitrogen and usable. The peptide presented by *Escherichia coli* preferably has a length of 3 to 15 residues.

Hereafter, a case where a target biomolecule is the NS3 protease will be mainly explained, but the biomolecule in the present invention is not limited to the NS3 protease. When the present invention is applied to another biomolecule, the NS3 protease and related items can be replaced with the target biomolecule and items therefor in the following description. The following explanation concerns a case where random peptide-presenting phage is used as a peptide library, but the peptide library used in the present invention is not limited to the random peptide-presenting phage.

First, to select a phage that binds to the target NS3 protease, the NS3 protease is adsorbed to, for example, a tube or a plate and the aforementioned library is brought into contact with the NS3 protease. Then, non-binding phages are washed away to leave the binding phages. After the washing, the remaining phages are eluted with an acid solution of about pH 2 or the like. Then, the eluate is neutralized and *Escherichia coil* is infected with the phages to amplify the phages. By repeating this selection process for a plurality of times, a plurality of phages having affinity for the target NS3 protease are concentrated. Then, to obtain a single clone, *Escherichia coli* is reinfected with the phages and the bacterium is allowed to form colonies on an agar medium. Each colony is cultured in a liquid medium and then phages present in the medium supernatant are precipitated and purified with polyethylene glycol or the like. DNA is prepared from the obtained phages and the structure of the peptide can be obtained by analyzing the nucleotide sequence of the DNA.

As the method for preparing a peptide library having random amino acid sequences, chemically synthesized peptides can also be used in addition to the above-described method using phages. As examples of such a method, there have been reported the method using beads (Lam, K.S. et al., Nature, 354, 82, 1991), the liquid phase focusing method (Houghton, R.A. et al., Nature, 354, 84, 1991), the microplate method (Fodor, S.P.A. et al., Science, 251, 767, 1991) and so forth. As protection groups such as one for an amino group in the peptide synthesis and condensing agents for condensation reaction, for example, those described in "Protein Engineering: Fundamentals and Applications", Ed. by Suzuki, K., Maruzen Co., Ltd., 1992; "Peptide Synthesis", Bodanszky M., et al, John Wiley & Sons, N.Y. 1976 and "Solid Phase Peptide Synthesis", Stewart, J.M. et al., W.H. Freeman and Co., San Francisco, 1969 and so forth can be used. For the solid phase method, various commercially available peptide synthesizers can be used.

The method for preparing peptides used in the present invention is not limited to the aforementioned chemical or biological methods.

To determine whether the obtained peptides inhibit the NS3 protease activity, for example, a peptide that can be digested by the NS3 protease as a substrate is prepared by a recombination method or a chemical synthesis method, labeled with fluorescence, radioisotope or the like and mixed with the NS3 protease in the presence of the peptide to be evaluated. Degree of inhibition for the digestion of the substrate compared with the case where the peptide to be evaluated is not added can be determined by examining the digestion of the substrate through liquid chromatography or electrophoresis.

Specifically, a gene coding for the amino acid sequence (985th to 1647th amino acid residues) in a polyprotein encoded by the HCV genome can be expressed and digestion of the substrate performed by using the obtained NS3 protease can be detected by liquid chromatography according to the method of Kakiuchi et al. (Biochem. Biophys. Res. Commun., 210, 1059, 1995).

Phages having affinity for the NS3 protease can also be screened by the method of Smith et al. (Smith G.P. et al., Methods in Enzymology, 217, 228-257). That is, a protein containing the catalytic domain of the NS3 protease obtained by expressing the gene coding for the NS3 protease as described above can be immobilized on a well of a microtiter plate and phages binding to the NS3 protease can be detected.

More precisely, DNA coding for the catalytic domain of the NS3 protease (catalytic region protein) is obtained based on the information of the HCV genome sequence. Desired DNA can be easily obtained by the PCR (polymerase chain reaction) method. At this time, a specific protein is preferably fused to the N-terminus or C-terminus of the protein to simplify the purification. As such a protein, the maltose-binding protein (MBP) or the like can be mentioned.

For example, since the NS3 protease is expressed in a patient infected with human hepatitis C virus, a sense chain oligonucleotide primer and an anti-sense chain oligonucleotide primer can be prepared based on the amino acid sequence coding for the region having the NS3 protease activity, and a DNA coding for the catalytic region protein of the NS3 protease can be amplified by PCR using these primers and cDNA prepared from a sample derived from the patient as a template. Alternatively, the DNA can also be obtained by total chemical synthesis. Further, when MBP is added, for example, there can be used a primer obtained by ligating an anti-sense DNA of the DNA coding for MBP to the sense primer of the catalytic region protein of the NS3 protease so that their reading frames match each other.

Thus, a DNA fragment coding for the catalytic domain of the NS3 protease, for example, a DNA fragment coding for the NS3 protease catalytic region protein fused with MBP (hereafter, also abbreviated as "MBP-NS34a" in the present specification) can be obtained and a recombinant vector for expressing the DNA can be prepared by incorporating the DNA fragment into a vector. The kind of the vector is not particularly limited so long as it can express the DNA in animal cells, plant cells, microorganism cells or the like. As for the MBP-fused protein, a substantial amount of the protein can be obtained by using a commercially available kit (for example, Protein Fusion and Purification System, New England Biolabs) to express the target protein according to the protocol attached to the kit.

The protein expressed in the obtained culture, preferably MBP-NS34a, can be separated and purified by column chromatography using a carrier on which a ligand having affinity for the protein is immobilized or the like. In case of MBP-NS34a, the expressed protein can be easily purified by column chromatography using a carrier on which amylose is immobilized, for example, Amylose Resin (New England Biolabs) or the like. Subsequently, the protein expressed as described above can be immobilized on a well of microtiter plate.

As the method for selecting a phage clone that specifically binds to the protein by using the immobilized MBP-NS34a or the like, for example, the method of Smith et al. (Smith G.P. et al., Methods in Enzymology, 217, 228-257) can be mentioned. For example, a phage peptide library is added to MBP-NS34a immobilized on a well of a microtiter plate, and the plate is incubated at a room temperature and washed sufficiently to remove non-specific binding phage. Then, a phage binding to the immobilized MBP-NS34a can be eluted by using a strong acid. By neutralizing the phage with alkali and infecting it into *Escherichia coli*, a phage clone specifically binding to MBP-NS34a can be amplified.

The phage can be obtained from a culture supernatant obtained by infecting the infected *Escherichia coli* with a M13KO7 helper phage and culturing it overnight, by precipitation with polyethylene glycol. By repeating similar screening with the amplified phage, for example, a phage that firmly binds to MBP-NS34a can be concentrated.

A phage that may bind to MBP-NS34a, for example, can easily be prepared by culturing an *Escherichia coli* colony on an ampicillin plate obtained by the above method and infecting the bacteria with a helper phage such as M13KO7. For example, a phage can be prepared by culturing an *Escherichia coli* colony on the ampicillin plate, infecting the bacteria with a helper phage such as M13KO7, culturing them overnight, centrifuging the culture solution and subjecting the supernatant to precipitation with polyethylene glycol. Whether the obtained phage clone binds to, for example, MBP-NS34a or not can be determined by the ELISA method utilizing an antibody recognizing that phage. For example, a phage clone coding for a peptide having affinity for MBP-NS34a can be finally obtained by using the HRP/anti-M13 conjugate (Amersham Pharmacia Biotech).

From the phage clone that has been thus confirmed to bind to MBP-NS34a, a double-stranded DNA derived from the selected phage can be obtained by using a FlexiPrep Plasmid extraction Kit (Amersham Pharmacia Biotech) or the like. For example, a random DNA region can be determined from the phage DNA that has been confirmed to bind to MBP-NS34a by the method of Sanger et al. (Sanger, F. et al., Proc. Natl. Acad. Sci. USA, 74, 5463, 1977), for example. The amino acid sequence specifically presented by the phage clone can be easily deduced from the sequence of this DNA region.

Standard operations required for handling of phage, DNA, and *Escherichia coli* as a recombination host described in the present specification are well known to those skilled in the art and described in, for example, the laboratory manual of Maniatis et al. (Maniatis T. et al., Molecular Cloning A Laboratory Manual, Cold Spring Harbor Laboratory, 1989). As all the enzymes and reagents to be used, commercially available products can be used. Usually, the products can be used according to their specified conditions to completely fulfill their purposes. In addition to the above general explanations, methods for biologically preparing peptides used in the present invention are specifically explained in detail in the examples of the present specification. However, the method for biologically preparing peptides used in the present invention is not limited to these methods.

A peptide including substitution, insertion and/or deletion of one or more amino acid residues in the amino acid sequence can be easily prepared by using a DNA coding for the peptide specified by the aforementioned amino acid sequence. For example, such a peptide can be obtained by mutating a transformant such as *Escherichia coli* to which a recombinant vector containing the DNA is introduced, by using an agent such as N-nitro-N'-nitro-N-nitrosoguanidine and collecting a gene DNA from microbial cells. The DNA may also be directly treated with an agent such as sodium nitrite. Furthermore, for example, the site-directed mutagenesis (Kramer, W. and Frits, H.J., Methods in Enzymology, 154, 350, 1987), the recombinant PCR method described in "PCR Experiment Manual", 155-160, HJB Publications, 1991, the method for preparing mutant genes by PCR described in "Jikken Igaku", Special Issue, 8 (9), 63-67, Yodosha Co., Ltd., 1990 and so forth can be used.

A substance inhibiting an interaction between the NS3 protease and a peptide, that is, a substance that interacts with the NS3 protease, can be screened by using the phage peptide obtained as described above. That is, a compound inhibiting binding of the phage peptide and the NS3 protease can be identified as follows. The NS3 protease is immobilized on, for example, a 96-well multi-titer plate or the like and various compounds and phage peptides appropriately prepared are each added to each well of the plate, and incubated. After washing, the amount of the phage left in each well is detected by using, for example, a horseradish peroxidase-conjugated goat anti-M13 antibody to identify a compound inhibiting binding of each phage peptide and the NS3 protease. Alternatively, if each phage peptide is labeled with a lanthanide compound such as europium in advance, the operation becomes simpler, and it is suitable for high throughput screening of many kinds of compounds according to the present invention. The phage peptide may also be directly labeled with a labeling substance.

As described above, it is preferable to label a recombinant organism or a peptide presented thereby with a labeling substance in the present invention. The labeling scheme may be direct labeling or indirect labeling through binding of a substance that is directly labeled with a labeling substance and specifically recognizes the recombinant organism or the like. A target biomolecule may also be labeled.

Examples of the labeling substance include radioisotopes, fluorescent substances, enzymes, biotin, avidin, streptavidin and lanthanide compounds such as europium and so forth. Examples of the radioisotopes include ³H, ¹⁴C, ³²P, ³⁵S, ¹²⁵I and so forth. Examples of the fluorescent substances include FITC, TRITC, DTAF and so forth. Examples of the enzymes include peroxidase, β-galactosidase, alkaline phosphatase and so forth. For labeling and detection using these labeling substances, the conventional ELISA method known to those skilled in the art can be used.

When it is confirmed that a substance inhibiting an interaction between a target biomolecule and a surrogate peptide selected as described above can regulate the activity of the biomolecule, the substance can be a lead compound used as a starting material in development of drugs.

Substances selected by the present invention can be made into substances having a higher biological activity by modifying them with various methods.

Specific examples of the peptide that binds to the NS3 protease include the peptides specified by the amino acid sequences of SEQ ID NOS: 1 to 12 obtained in the examples and the corresponding peptides having a cyclic structure, in which two cysteine residues contained in each amino acid sequence forms a disulfide-bond. In the above sequences, the amino acid residue may be either an L- or D-amino acid residue, preferably an L-amino acid residue. The peptides according to the present invention have two cysteine residues, which may form a disulfide-bond to form a cyclic structure. Such cyclic peptides are of course encompassed in the scope of the peptides used in the present invention. Although the present invention is not bound by any specific theory, but it is possible that the above-described cyclic structure is essential for the peptides used in the present invention to express a desired physiological activity. Therefore, in such a case, the peptide having the cyclic structure is a particularly preferred embodiment in the present invention.

A peptide including substitution, insertion, and/or deletion of one or more amino acid residues in any of the aforementioned amino acid sequences and having affinity for the NS3 protease or a corresponding peptide having a cyclic structure, in which two of the cysteine residues contained in the peptide form a disulfide bond, are also provided by the present invention. The kind of one or more substituted and/or inserted amino acids is not particularly limited, but an L-amino acid is preferred. Whether a peptide including substitution, insertion and/or deletion of one or more amino acid residues substantially has affinity for the NS3 protease or not can easily be confirmed by those skilled in the art according to the methods described in the examples.

According to yet another aspect of the present invention, there are provided a drug containing as an active ingredient any of the above peptides or substances which are selected by the method of the present invention using these peptides and which interact with the NS3 protease, preferably a drug in the form of a pharmaceutical composition containing any of the above peptides or the interacting substances and additives for pharmaceutical preparations. Since these peptides or the interacting substances can inhibit the NS3 protease activity, the aforementioned drug is useful for treatment, prevention or diagnosis of diseases of mammals including humans induced by abnormality of liver cells due to infection and proliferation of hepatitis C virus, particularly, diseases accompanied with degeneration of liver cells. Examples of such diseases include cirrhosis, liver cancer and so forth, but the diseases are not limited to these ones.

One or more kinds of peptides selected from the above peptides may be used as they are as the above drug. However, it is usually preferred that a pharmaceutical composition containing one or more kinds of the above peptides as an active ingredient is prepared by using one or more kinds of pharmaceutically acceptable additives for pharmaceutical preparations, and used for treatment and/or prevention of the aforementioned diseases. In view of pharmacokinetics such as solubility, absorption and excretion and/or formulation methods, the peptides may be in the form of a physiologically acceptable salt. Examples of administration routes of the pharmaceutical composition include systemic administration such as intravenous administration, intrarectal administration and oral administration, as well as local administration such as external use, instillation of eye drop, nasal drop or ear drop, and local injection.

For example, an agent for systemic administration such as injection for intravenous administration or drip infusion is a preferred form of the pharmaceutical composition. Use of pharmaceutical compositions containing the active ingredient encapsulated in liposomes or the like or bound to antibodies or the like may improve affinity or selectivity for a specific target organ. However, as a matter of course, the administration route can be appropriately selected depending on the kind of the applicable disease, purpose of administration including treatment or prevention, conditions of patients and so forth, and a dosage form suitable for each administration route can also be appropriately selected.

### Examples

The present invention will be explained in further detail with reference to the following examples, but the scope of the present invention is not limited to these examples.

### Example 1: Preparation of phage library and selection of specifically binding phage peptide by panning method

This example shows a method for preparing a phage library and a method for identifying a phage expressing a peptide binding to a protein in a directed manner, which is selected by screening a library by using the panning method.

### <1> Construction of random peptide-presenting vector pSBSX

A phagemid vector for presenting a single-stranded antibody, pCANTAB5E (available from Amersham Pharmacia), was modified into a vector for presenting random peptides by using synthetic oligonucleotides. As the reagents and enzymes, commercially available products were used. As the synthetic DNA primers, products manufactured by Japan Bio Service were used. Two kinds of oligonucleotides shown below were annealed and inserted into the *Nco*I and *Not*I sites of the pCANTAB-5E vector to prepare the pSBSX vector. Sense strand: 
5′-CATGGCAGATCTTTAAGTCGACTCTAGAGGCCTCTGC-3′ (SEQ ID
NO: 13)
 Anti-sense strand:

5′-GGCCGCAGAGGCCTCTAGAGTCTACTTAAAGATCTGC-3′ (SEQ ID
NO: 14)

### <2> Preparation of vector DNA

A phage random peptide library was prepared by using the pSBSX vector prepared as described above as a raw material, according to the method of Nishi et al. (Jikken Igaku, 11, 13, 1759-1764), the method of Smith et al. (Smith G.P. et al., Science, 249, 386-390, 1990) and the method described by Koivunen et al. (see Koivunen et al., 1995, supra and Koivunen et al., 1994b, supra). The plasmid pSBSX was prepared as follows. *Escherichia coli* XL2-Blue transformed with pSBSX was inoculated into 1 L of 2 x YT-AG medium (2 x YT medium containing 100 µg/ml ampicillin and 2% glucose) and cultured overnight at 37°C with shaking. The plasmid pSBSX was prepared from the obtained microbial cells by using a Qiagen Plasmid Maxi Kit (QIAGEN), which can purify plasmids of high purity. About 1.5 mg of the plasmid was obtained from 1 L of the culture.

Subsequently, 30 µg of the pSBSX DNA was digested with 120 units each of *Nco*I, *Bgl*II, *Sal*I and *Not*I (Takara Shuzo) at 37°C for about 16 hours. The reaction mixture was subjected to phenol/chloroform extraction and chloroform extraction, and DNA was precipitated with ethanol from the aqueous phase. Then, the obtained DNA was dissolved in sterilized water. After sufficient digestion with the enzymes was confirmed by agarose gel electrophoresis and self-ligation, the plasmid DNA was purified by using Sephacryl S-400 (Amersham Pharmacia Biotech).

### <3> Degenerate oligonucleotides coding for random peptides

Degenerate oligonucleotides (a collection of random DNAs coding for random peptides) were prepared for each library according to the method of Smith et al. (supra) for preparing double-stranded DNA by PCR using 5' end biotinylated primers. Accordingly, 10 kinds of libraries coding for peptides designated as X₆, X₉, X₁₅, CX₄C, CX₅C, CX₆C, CX₇C, CX₈C, CX₉C and CX₁₂ were prepared. Here, "C" represents cysteine, and "Xₙ" indicates that a given number (n) of independently selected amino acids are serially linked. These libraries can each present a cyclic peptide when the peptide contains at least two cysteine residues. The oligonucleotides were constructed such that "C" should be encoded by the codon TGT and that "Xₙ" should be encoded by (NNK)ₙ. Here, "N" is an equimolar mixture of A, C, G and T, and "K" is an equimolar mixture of G and T. The collection of DNAs represented by the NNK includes 32 kinds of combinations, which include codons of all the 20 kinds of amino acids. Degenerate oligonucleotides are synthesized by repeating this NNK the same number of times as the desired number of amino acids.

The peptides are not only presented in a straight-chain form (in an unconstrained or linear form), but also can be expressed in a form having a loop structure (in a constrained or loop form) by providing two cysteine residues at both ends of the sequence to form a disulfide bond. Accordingly, the peptide represented by CX₅C can be expressed by an oligonucleotide having the sequence TGT(NNK)₅TGT.

PCR amplification was performed by using 5 µg of a synthetic oligonucleotide with a biotinylated 5' end, 5'-ACTCGGCCGACGGGGC-3' (SEQ ID NO: 15), and 5 µg of non-labeled synthetic oligonucleotide,
5'-TTCGGCCCCAGCGGCCCC-3' (SEQ ID NO: 16), as primers and 1 µg of synthetic oligonucleotide,
5'-ACTCGGCCGACGGGGCT(NNK)ₙGGGGCCGCTGGGGCCGAA-3' (n = 4 to 15) (SEQ ID NO: 17), as a template to obtain a double strand. Taq DNA polymerase (Takara Shuzo) and an attached reaction buffer were used for PCR. A cycle composed of 95°C for 2.5 minutes, 50°C for 4 minutes and 72°C for 2.5 minutes was repeated five times in total to perform amplification and treatment at 72°C for 5 minutes was finally conducted. This PCR product was precipitated with ethanol and the obtained DNA was treated with 200 units each of *Nco*I and *Not*I (Takara Shuzo) at 37°C for 16 hours. Then, the biotinylated DNA fragment was removed by using Streptavidin Agarose (GIBCO BRL). The solution was subjected to phenol/chloroform extraction and chloroform extraction and DNA was precipitated with isopropanol. The obtained DNA was dissolved in sterilized water to obtain random insert DNA.

### <4> Ligation of phagemid vector DNA and random insert DNA

The random insert DNA was ligated to the DNA coding for the gene III protein in the pSBSX vector, which was designed such that the peptide encoded by the random insert DNA should be present at the N-terminus of the gene III protein as a fusion protein upon expression of gene III. 7.45 µg of the pSBSX DNA digested with *Nco*I, *Bgl*II, *Sal*I and *Not*I obtained in <2> and 0.2 µg of the random insert DNA obtained in <3> were subjected to ligation reaction at 16°C for 16 hours by using an isovolume Ligation High Mix (Toyobo). The reaction mixture was subjected to phenol/chloroform extraction and chloroform extraction and DNA was precipitated with ethanol. The obtained DNA was dissolved in sterilized water and then subjected to ultrafiltration by using a Millipore Filter Ultrafree C3 (trade name; Millipore) to be concentrated to a DNA concentration of 5 µg/µl or higher. The obtained solution was stored at -20°C until it was used for preparation of a phage random peptide library.

### <5> Preparation of phage display random peptide library

Phages were prepared by using the *Escherichia coli* TG1 strain, according to the protocol attached to the product manufactured by Amersham Pharmacia Biotech. *Escherichia coli* was transformed by electroporation. As *Escherichia coli* competent cells, those of the commercially available TG1 strain for electroporation (Amersham Pharmacia Biotech) were used. A required amount of competent cells were thawed on ice and mixed with 2 µl of the ligation reaction mixture obtained in <4> per 200 µl of the competent cells. This mixture was transferred into a sufficiently cooled cuvette (BIO-RAD Inc.) having a gap width of 0.2 cm and electrical pulses (4.5 ms) were applied thereto by using a Gene Pulser™ electroporation apparatus (BIO-RAD) under conditions of 2500 V, 200 Ω and 25 µF. The treated *Escherichia coli* was transferred to SOC medium (20 g/L Bacto trypton, 5 g/L yeast extract, 0.5 g/L NaCl, 10 g/L glucose) in an amount 9 times as much as that of the *Escherlchia coli*, which was incubated at 37°C in advance, and cultured at 37°C for 30 minutes with shaking.

This culture solution was appropriately diluted, plated on an SOB agar medium plate (SOBAG plate; 20 g/L Bacto trypton, 5 g/L yeast extract, 0.5 g/L NaCl, 10 mM MgCl₂, 20 g/L glucose and 15 g/L Bacto agar) containing 20 µg/ml of ampicillin (Wako Pure Chemical Industries) and 50 µg/ml of kanamycin (Wako Pure Chemical Industries) and cultured overnight at 37°C as standing culture. The number of colonies formed on the plate surface was counted to obtain the original library size. It was estimated that this phage culture solution contained about 1 x 10⁹ clones in total.

### <6> Collection of phage particles

The remaining culture solutions for 10 times of the above-described transformation were transferred together to 200 ml of 2 x YT-AG medium maintained at 37°C. The M13K07 helper phage at an m.o.i. of 10 was added thereto and the mixture was further shaken at 37°C and 150 rpm for 30 minutes. The microbial cells were collected by centrifugation (4°C, 3000 rpm, 15 minutes), resuspended in 400 ml of 2 x YT-AK medium (2 x YT medium containing 100 µg/ml of ampicillin and 50 µg/ml of kanamycin) and cultured at 37°C overnight with shaking at 150 rpm. The culture solution was subjected to centrifugation (4°C, 3000 rpm, 30 minutes) and phage was obtained at a high concentration from this phage culture solution by the method utilizing precipitation with polyethylene glycol (PEG). A PEG/NaCl solution (20% PEG8000 (W/V)/2.5 M NaCl) was added thereto in a 0.15-fold amount and the mixture was left standing overnight at 4°C. Then, the supernatant was removed by centrifugation (4°C, 16000 rpm, 30 minutes) and the precipitate was resuspended in TBS (50 mM Tris-HCl, 150 mM NaCl) buffer in a 0.1-fold amount. The supernatant was collected by centrifugation (4°C, 16 rpm, 15 minutes) and phage particles were precipitated again with the PEG/NaCl solution in a 0.15-fold amount and suspended in the TBS buffer to finally concentrate the phage solution about 100-fold.

### <7> Measurement of phage titer

The titer of the obtained phage was calculated in terms of TU (transducing unit)/ml, which is a unit of ampicillin resistance, as described below and the phage was used to screen a peptide binding to a biomolecule. Colonies of the *Escherichia coli* TG1 strain were cultured overnight in 5 ml of 2 x YT medium, and 0.2 ml of this culture solution was cultured at 37°C for about 3 hours by using 20 ml of 2 x YT medium. 100 µl of this *Escherlchia coli* culture solution and 2 µl of the appropriately diluted phage solution were mixed in a tube, and the mixture was left at room temperature for 10 minutes. This mixture was plated on an SOBAG plate containing 40 µg/ml of ampicillin and cultured at 37°C overnight. The number of colonies on the plate was counted and the titer of the phage for generating one colony on the plate was defined as 1 TU/ml.

### <8> Construction of plasmid expressing HCV NS3 protease

The DNA fragment coding for the region having the HCV NS3 protease activity (including 979th to 1710th amino acids) was prepared by making chemically synthesized oligonucleotides double-stranded and successively ligating these oligonucleotides. The HCV genome sequence is registered at, for example, GenBank as HPCJCG (Accession Number D90208) and any sequence information can be utilized.

Subsequently, the DNA coding for the region having the NS3 protease activity was introduced into the vector pMAL-c2 expressing MBP fusion protein (New England Biolabs) so that their reading frames should match, to construct a recombinant vector for expressing the NS3 protease protein. Use of the pMAL bacterial expression vector for the purpose of expressing the MBP fusion protein is well known to those skilled in the art. Subsequently, the obtained DNA was introduced into the *Escherichia coli* XL2-Blue strain (Stratagene) by the calcium chloride method. A strain in which the insertion occurred so that the fusion protein could be expressed, was selected from the obtained transformants.

### <9> Expression in large amount and purification of MBP-NS34a

The MBP fusion protein was expressed and purified according to the protocol attached to the aforementioned expression vector. Colonies of the *Escherichia coli* strain prepared as described above formed on LB agar medium containing 100 µg/ml of ampicillin were directly inoculated into five of 500-ml Sakaguchi flasks, each containing 200 ml of LB medium containing 50 µg/ml of ampicillin, that is, 1 L in total, and shaken at 30°C for 14 to 16 hours to be cultured until OD600 reached 0.5 to 0.7. The medium was cooled at 4°C, and IPTG (isopropyl-β-D-thiogalactopyranoside) was added at a final concentration of 0.5 mM. The culture was further cultured at 20°C for 4 hours with shaking. Then, the microbial cells were collected by centrifugation. The microbial cells were washed with 0.85% NaCl solution and suspended in 50 ml of Buffer A (10 mM Na-phosphate buffer (pH 7.2), 30 mM NaCl, 2 mM DTT (dithiothreitol)). After the solution was frozen and thawed, a supernatant was collected by centrifugation. The collected supernatant was subjected to precipitation with 70% ammonium sulfate and a pellet was collected by centrifugation. The pellet was suspended in Buffer B (10 mM Na-phosphate buffer (pH 7.2), 30 mM NaCl, 10 mM 2-ME (mercaptoethanol), 0.25% Tween 20) and left overnight at 4°C to obtain a crude extract. Subsequently, the extract was applied to an amylose resin column (15 mm in diameter and 10 cm in length) equilibrated with Buffer B, at a flow rate of 0.5 ml/min. This process was repeated several times.

The column was washed with Buffer B of 4 times the column volume and Buffer C (10 mM Na-phosphate buffer, pH 7.8, 0.5 M NaCl) of 8 times the column volume and eluted with Buffer C containing 10 mM maltose to recover a purified enzyme solution. After quantification, glycerol at a concentration of 20% was added to the solution and the mixture was stored at-80°C until use. About 10 mg of purified MBP-NS34a could be obtained from 1 L of the culture.

### <10> Quantification of MBP-NS34a by ELISA

1.2 µg/ml of goat anti-MBP antibodies (New England Biolabs) diluted in 100 µl of PBS(-) (phosphate-buffered physiological saline not containing divalent metal ions) was introduced into wells of a microtiter plate and left standing at room temperature for 8 hours or longer to coat the wells with the antibodies. Each well was washed with 200 µl of PBS(-). Then, 1% BSA (bovine serum albumin) dissolved in 200 µl of PBS(-) was added thereto and allowed to react at room temperature for 8 hours or longer to block the wells. Subsequently, each well was washed with 200 µl of PBS(-) containing 0.05% Tween 20. Then, 100 µl of the purified MBP-NS34a solution was added thereto and incubated at room temperature for 2 hours or longer.

Each well was washed with 200 µl of PBS(-) containing 0.05% Tween 20. Then, 100 µl of horseradish peroxidase-conjugated goat anti-MBP antibodies (New England Biolabs) diluted 50,000 times with PBS(-) was added thereto and incubated at room temperature for 2 hours or longer. Each well was washed with 200 µl of PBS(-) containing 0.05% Tween 20. Then, 100 µl of ABTS (2,2'-azino-di-(3-ethyl-benzthiazoline-sulfonic acid)) solution (Amersham Pharmacia Biotech) was added thereto and incubated at room temperature for 5 minutes. 100 µl of 1 M NaOH (Wako Pure Chemical Industries) was added thereto to stop the reaction. The optical density at 450 nm of the reaction mixture was measured and the MBP-NS34a was quantified as the MBP equivalent based on a calibration curve. The calibration curve was prepared by performing similar ELISA using MBP (New England Biolabs) having known concentrations in a range of 20 to 300 ng/ml.

### Example 2: Screening of MBP-NS34a-binding phage by panning method

The panning method was performed basically according to the method of Smith et al. (Methods in Enzymology, 217, 228-257) and the protocol attached to a phage display system manufactured by Pharmacia. Maltose was added at a final concentration of 10 mM to the MBP-NS34a protein and the phage library.

### <1> Coating of polystyrene tube with target protein

The protein (MBP-NS34a) solution used for panning and ELISA was prepared at a concentration of 10 µg/µl by using a protein coating buffer (10 mM NaHCO₃ buffer, pH 9.6). A 6-ml polystyrene tube (Falcon 2063) was filled with about 5 ml of the protein solution and coated with the protein on a PALM REACTION PR-12 (NEWCO) overnight at 4°C. The protein solution was removed and the tube was washed once with TBS buffer. Then, the tube was filled with about 5 ml of a blocking solution (10 mM NaHCO₃ buffer containing 5% BSA) and shaken on the PALM REACTION PR-12 at 4°C for 2 hours. The blocking solution was removed and the tube was washed with TBST (TBS containing 0.5% Tween 20) buffer six times. Then, a predetermined amount of phage library (about 2 x 10¹² TU) prepared with TBST buffer containing 1 mg/ml (0.1%) of BSA was added and the tube was shaken on the PALM REACTION PR-12 overnight at 4°C.

### <2> Washing and elution of phage

The phage solution was removed from the tube by using a pipette and the tube was washed with TBST buffer 10 times. 4 ml of elution buffer (0.1 N glycine-HCl containing 0.1% BSA, pH 2.2) was filled and the tube was shaken on the PALM REACTION PR-12 for 15 minutes to elute the phages binding to the tube, and the eluate was neutralized with 400 µl of 2 M Tris solution (pH unadjusted).

### <3> Reinfection and rescue of phage and measurement of collected phage

One of colonies of the *Escherichia coli* TG1 strain grown on a minimum medium agar plate was cultured overnight at 37°C in 5 ml of LB medium by using a shaking incubator. 0.2 ml of the TG1 culture solution was cultured with shaking in 20 ml of 2 x YT medium at 37°C for about 3 hours to obtain a culture solution having an OD of about 1.0, and it was left standing at room temperature for 5 minutes. A half amount of the eluted phage solution collected in the above <2>, i.e., 2.2 ml, and an equal volume of the TG1 culture solution were mixed and shaken at 37°C for 15 minutes. A part of the mixture was streaked on an SOBAG plate and incubated overnight at 37°C. The number of collected phage clones was calculated and templates for sequencing were prepared. Microbial cells were collected from the remaining mixture by centrifugation (4°C, 3000 rpm, 15 minutes), resuspended in 2 x YT-AK medium containing 40 mg/ml of kanamycin and 40 mg/ml of ampicillin and cultured at 37°C overnight with shaking.

Similarly, the culture solution was subjected to precipitation with polyethylene glycol to collect the amplified phages. The culture solution was collected by centrifugation (4°C, 10,000 rpm, 10 minutes), and PEG/NaCl solution was added in a 0.15-fold volume. The mixture was left standing at 4°C for 4 hours or longer. Then, the mixture was centrifuged again (4°C, 10000 rpm, 10 minutes), and the precipitate was suspended in 1 ml of TBS buffer. Finally, centrifugation (4°C, 3000 rpm, 15 minutes) was performed and the supernatant was collected into a 1.5-ml Eppendorf tube so that insoluble matters were not involved. The titer of the phage was measured according to the phage titration method.

The phage clones binding to MBP-NS34a were concentrated by repeating the above operations of <1> to <3> three to four times. The second and subsequent cycles were performed according to <1> and <2> by using phages (10¹¹-10¹² TU/ml) obtained by amplifying the phages collected in each previous screening. Phages presenting peptides showing affinity for MBP-NS34a were included in the concentrated phages, and it was considered that the peptides were encoded by random DNA corresponding to them.

### <4> Phage clones potentially binding to MBP-NS34a and preparation of phage DNA

Phage clones and double-stranded DNA were prepared from the *Escherichia coli* TG1 colonies obtained on the SOBAG agar plate used in the above <3>. The obtained colonies on the plate were cultured overnight in 2 x YT-AG medium containing 40 µg/ml ampicillin. 50 µl of the culture solution was transferred into a tube to prepare the phage. Sequencing templates were prepared by using the remaining 950 µl. To 50 µl of the culture solution, the M13K07 helper phage at an m.o.i. of 10 and 500 µl of 2 x YT-AG medium were added, and the mixture was shaken at 37°C for 30 minutes. Microbial cells were collected by centrifugation (4°C, 3000 rpm, 15 minutes), resuspended in 1 ml of 2 x YT-AK medium and cultured overnight at 37°C with shaking. The following day, the culture solution was collected in an Eppendorf tube by centrifugation (4°C, 3000 rpm, 15 minutes). Further, when it was considered that there were only a small number of phage particles, a concentrated phage solution was prepared by using the PEG/NaCl solution in a 0.15-fold amount. These phages were assumed to be phage clones potentially binding to MBP-NS34a and the binding property was determined according to the ELISA method described below. The template DNA for sequencing was prepared by using a FlexiPrep Plasmid Extraction Kit (Amersham Pharmacia Biotech) according to the attached protocol.

### <5> Identification of phage that binds to MBP-NS34a by ELISA method

The protein (MBP-NS34a) was immobilized on a 96-well microtiter plate (Maxisorp, Nunc) by using 50 µl of the solution per well in the same manner as in the aforementioned panning method (overnight at 4°C, 10 mM NaHCO₃ buffer, pH 9.6, protein concentration of 10 µg/ml). The plate was blocked by introducing 200 µl of 5 mg/ml (0.5%) BSA fraction V solution prepared by 10 mM NaHCO₃ per well and leaving it at 4°C for 1 hour as in the panning method. After rinsed with TBS buffer once, 50 µl of the phage solution prepared in <4> was added to each MBP-NS34a-immobilized well, and incubated overnight at 4°C. Each well was washed with TBS buffer four times, and 50 µl of horseradish peroxidase (HRP)-conjugated sheep anti-M13 phage antibodies (Amersham Pharmacia Biotech, Code No.27-9411-01) diluted 2000 times by using 5% skim milk was added per well and incubated at room temperature for 1 hour.

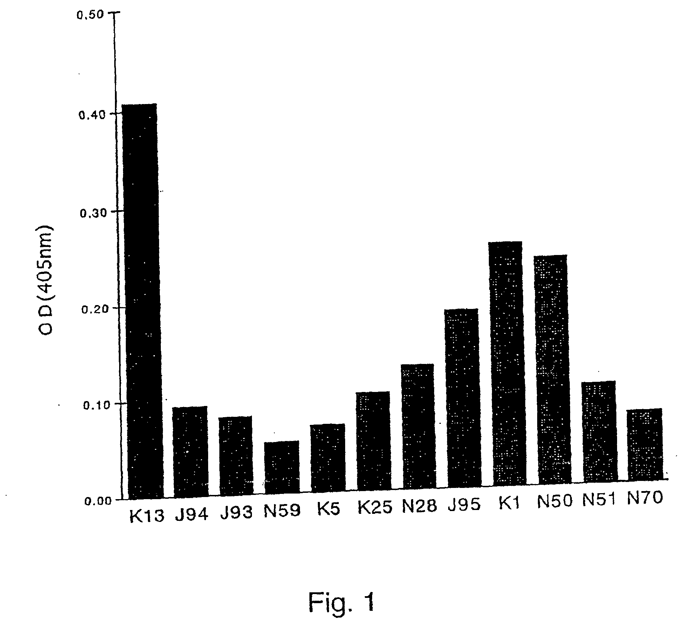

Each well was washed with 200 µl of TBS containing 0.5% Tween 20 five times. Then, 100 µl of color-developing substrate solution (50 mM citric acid containing 0.2 mg/ml ABTS, pH 4.0; Amersham Pharmacia Biotech, Code No. 27-9402-01) was added and further incubated at room temperature. The optical density was measured at 405 nm by a 96-well plate reader (BIO-RAD). When the optical density of MBP-NS34a was at least twice as high as the optical density obtained when MBP was used instead of MBP-NS34a, the solution was determined to contain a phage clone binding to MBP-NS34a. The affinity for the NS3 protease, of the phages having peptides selected according to the present invention was determined by the ELISA method and the results are shown in Fig. 1. As the negative control, the M13 phage that expressed no random peptide was used.

### <6> Analysis of nucleotide sequences of phage clones that bind to MBP-NS34a

Plasmid was prepared from 950 µl of culture solution by using a FlexiPrep kit (Amersham Pharmacia Biotech). 2.5 µl (about 100 ng) of DNA corresponding to the phage identified as described above among the phagemid DNA obtained in <4> and 1.6 pmol of sequencing primer 5'-TGAATTTTCTGTATGGGG-3' (SEQ ID NO: 18) prepared so that it should bind to a position about 60 nucleotide upstream from the random DNA region on the phage were mixed. A cycle sequencing reaction was performed by using a Prism DNA Cycle Sequencing Kit (PE Biosystems) and a PCR apparatus, PCR 9600 (Perkin-Elmer), according to the attached protocol. After the completion of the reaction, the reaction product was collected by ethanol precipitation and dissolved in formamide. Each DNA sequence was determined by using a model 377 DNA sequencer (ABI) and the amino acid sequence in the random region of the phage was analyzed. The amino acid sequences and appearance frequency are shown in Table 1.

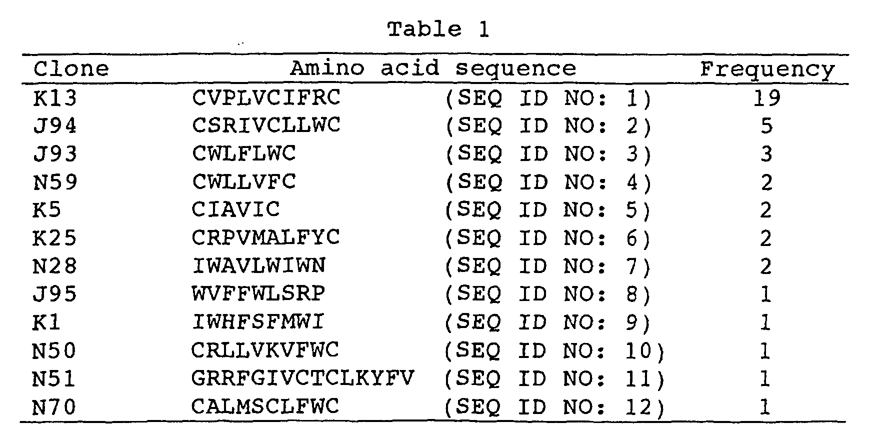

### <7> Preparation of synthetic peptides

The sequences of the peptides obtained from the phage peptide library are shown in Table 1. These peptides can have either a straight-chain structure or an intramolecular cyclic structure, in which two cysteine residues form a disulfide bond. Chemical synthesis of the peptides was entrusted to Sawady Technology. Various peptides having a straight-chain or cyclic structure were synthesized by the solid phase method and purified to 80% purity or higher by reverse phase HPLC.

### (Conditions of liquid chromatography)

Column: Kromasil 100 C18, 5 µm, 125 x 4 mm
Temperature: 20°C
Flow rate: 0.75 ml/min
Eluant: Buffer A = 0.1% trifluoroacetate aqueous solution
Solvent: 0.1% TFA (trifluoroacetate) aqueous solution/acetonitrile (20/80)

All the synthesized peptides were confirmed for their molecular weights by mass spectroscopy and used for evaluation. Sequences of the synthesized peptides are shown in Table 2.

### Example 3: Examination of protease inhibitory ability of synthetic peptides

### <1> NS3 protease inhibitory activity test using substrate cleavage reaction as index

An NS3 protease cleavage sequence between NS5a and NS5b in the HCV polypeptide was synthesized. The protease inhibitory ability of each of the synthetic peptides was examined according to the method of Kakiuchi et al., in which a synthetic substrate having an amino terminus labeled with a dansyl group was reacted in the presence of a protease and a cleavage degree is measured by reverse phase HPLC (Biochem. Biophys. Res. Commun., 210, 1059, 1995). 1 µg of the purified MBP-NS34a and a reaction mixture (50 mM Tris-HCl (pH 7.5), 30 mM NaCl, 5 mM MgCl₂ and 10 mM DTT) containing an evaluation sample prepared at an appropriate concentration were incubated at 25°C for 15 minutes. To the solution, the synthesized substrate was added at a final concentration of 50 µM, and the mixture was allowed to react at 37°C for 1 to 3 hours. Subsequently, the enzymatic reaction was stopped by adding a reaction-stopping solution (20% acetonitrile, 0.1% TFA) in a volume 9 times as much as the reaction volume. Then, the cleavaged peak of the synthesized substrate was detected (excited at 340 nm and measured at 510 nm) by a reverse phase column (YMC-AM-302) to calculate the cleavage rate when the inhibitor was added. Further, a value obtained by dividing this cleavage rate when the inhibitor was added by the cleavage rate when no inhibitor was added, was subtracted from 1 and then multiplied by 100. The obtained value was used as the inhibition rate (%) of the evaluated drug. In the above method, the peptide concentration inhibiting 50% of the protease activity (IC₅₀) was calculated (Table 2).

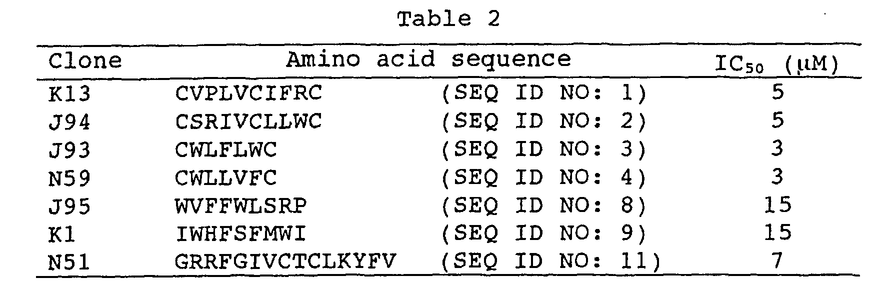

As a result, it was revealed that the above synthetic peptides had an activity for inhibiting the NS3 protease activity.

### <2> Inhibition of binding of phage peptide and MBP-NS34a by NS4a peptide, 4A18-40

A possibility that an action site of each phage peptide clone obtained as described above and that of the NS4a peptide derived from virus known to be activated by the binding to the NS3 protein were identical or overlapped was examined. A partial peptide of the NS4a peptide composed of the 18th to 40th residues from the amino terminus (referred to as 4A18-40 peptide) is known as a minimal region required to enhance the NS3 protease activity (Tanji et al., J. Virol., 69, 4017-4026, 1995). Accordingly, an influence on the binding of each phage peptide and the MBP-NS34a protein was analyzed by the phage ELISA method using a chemically synthesized 4A18-40 peptide (LTTGSVVIVGRIILSGRPAVVPD, SEQ ID NO: 19). 10 µg/ml of the MBP-NS34a protein to which the 4A18-40 peptide was added in an amount of 10 or 20 times in excess in a molar ratio was immobilized on each well of a 96-well microtiter plate. The plate was blocked by using 100 µl of 0.5% BSA solution prepared with 10 mM NaHCO₃, and rinsed once with 200 µl of TBS buffer. 100 µl of the phage solution prepared in Example 2, <4> was added to each well of the 96-well plate on which the NS4a peptide and the MBP-NS34a protein were immobilized, and incubated overnight at 4°C. Each well was washed with 200 µl of TBS buffer four times. Then, 50 µl of horseradish peroxidase (HRP)-conjugated sheep anti-M13 phage antibodies diluted 2000 times with 5% skim milk was introduced into each well and incubated at room temperature for 1 hour.

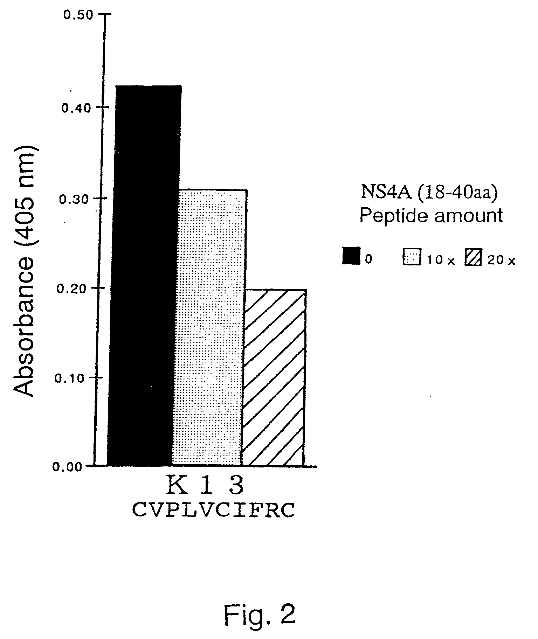

Each well was washed with 200 µl of TBS containing 0.5% Tween 20 five times. Then, 100 µl of a peroxidase color-developing substrate solution was added thereto and further incubated at room temperature. The optical density at 405 nm was measured by a 96-well plate reader (BIO-RAD). The optical density due to binding of the MBP-NS34a and the phage peptide observed in the presence of the NS4a peptide was measured. As a result, binding of the phage clone K13 to the MBP-NS34a protein was increasingly inhibited as the abundance ratio of the 4A18-40 peptide rose to 10-fold and 20-fold (Fig. 2).

### <3> Screening of inhibitor for binding of phage peptide and MBP-NS34a

A binding inhibitor was screened by using the above phage ELISA system. As peptides used for screening, the phage peptides whose protease inhibitory activity was confirmed by using the synthetic peptides were prepared in an amount required for screening according to the method of Example 2 <3>. Each well of a 96-well microtiter plate was coated by using 50 µl of the MBP-NS34a protein solution prepared at 10 µg/ml. The plate was blocked by using 100 µl of 0.5% BSA solution prepared with 10 mM NaHCO₃ and rinsed once with 200 µl of TBS buffer for each well. A mixed solution of a compound in a chemical library (synthesized in our company) diluted to an appropriate concentration and each phage prepared as described above was prepared, and the mixture was added to each well coated with the MBP-NS34a protein in an amount of 100 µl and incubated overnight at 4°C. Each well was washed with 200 µl of TBS buffer four times. Then, 50 µl of horseradish peroxidase (HRP)-conjugated sheep anti-M13 phage diluted 2000 times with 5% skim milk was introduced into each well and incubated at room temperature for 1 hour.

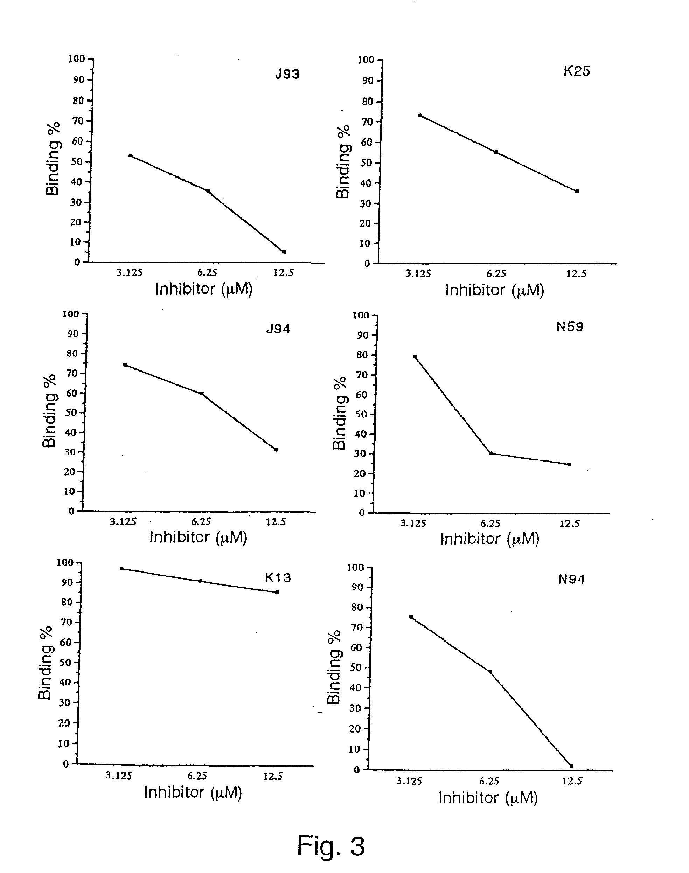

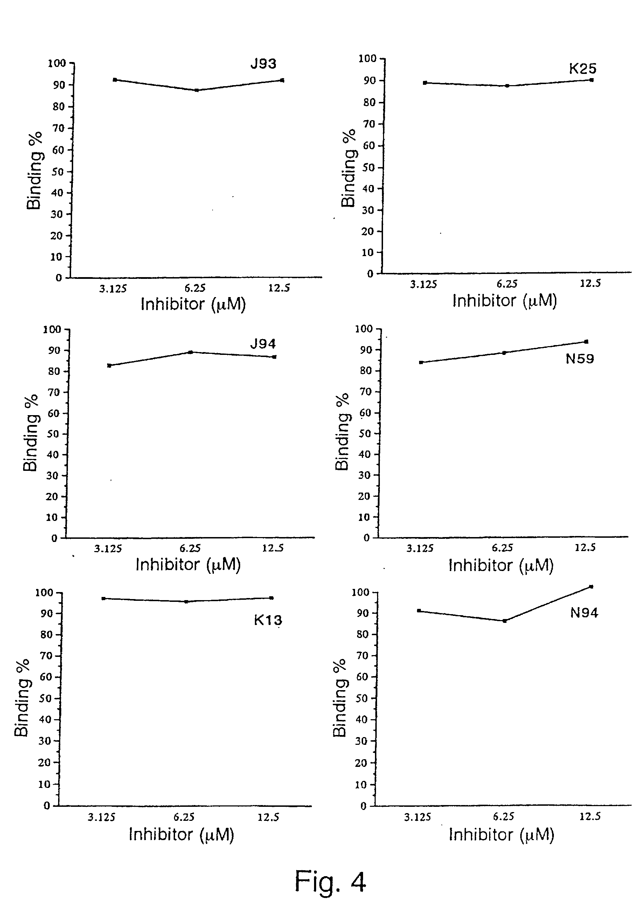

Each well was washed with 200 µl of TBS containing 0.5% Tween-20, five times. Then, 100 µl of ABTS solution was added thereto and further incubated at room temperature. The optical density at 405 nm was measured by using a 96-well plate reader (BIO-RAD). There was selected Compound HCP1271 (Compound 5 described in Japanese Patent Laid-Open (KOKAI) No. 2000-7645), in the presence of which the optical density by binding of the MBP-NS34a and the phage peptide was lower than the optical density observed in the absence of the compound. Further, this inhibitory compound was serially diluted and its influence on the binding of 5 kinds of other phage peptides, which inhibited the NS3 protease activity, was similarly determined by the phage ELISA method. As a result, HCP1271 concentration-dependently inhibited the binding of the MBP-NS34a and the phage peptide (Fig. 3). As a result of measurement of the protease activity inhibitory ability of this compound according to the method of Example 3, <1>, it was found that HCP1271 strongly inhibited the NS3 protease activity with an IC₅₀ value of 2.2 µM. Further, since a compound HCP1231 similar to HCP1271, was retrieved by the search of the chemical library, its NS3 protease activity inhibitory ability and influence on the binding of the phage peptide and the NS3 protease were examined. As a result, it showed very weak protease activity inhibitory activity and scarcely inhibited the binding of the phage peptide and the NS34a (Fig. 4). That is, it was shown that the compound obtained from screening of the inhibitor for binding of the phage peptide and the protease protein could inhibit the protease activity very well.

The structural formulae of the compounds HCP1231 and HCP1271 are shown below.

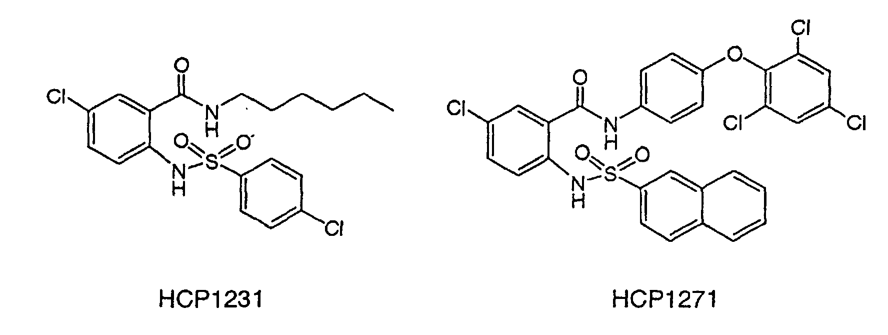

### Industrial Applicability

The method of the present invention is useful as a simple method for screening drugs inhibiting an interaction between various biological components such as an interaction between proteins or between protein and nucleic acid.

The peptide compound of the present invention has affinity for the HCV serine protease and is useful as an active ingredient of a preventive and/or therapeutic drug for diseases induced by abnormality of liver cells due to infection and proliferation of hepatitis C virus, for example, cirrhosis and liver cancer. Moreover, the screening method of the present invention is useful as a method for selecting a compound capable of modulating a protein function in search of therapeutic drugs for diseases.

## Claims

1. A method for screening a substance which interacts with a specific region of a biomolecule having an activity, to regulate the activity, said method comprising the following steps:
(a) a step of selecting a recombinant organism that interacts with the biomolecule, from a peptide library composed of a collection of recombinant organisms each presenting at least one of various peptides on its surface, with a proviso that the interaction is not an antigen-antibody reaction; and
(b) a step of selecting a substance inhibiting the interaction between the selected recombinant organism or a peptide presented by the recombinant organism and the biomolecule.

2. The method according to Claim 1, wherein the biomolecule is a protein, a nucleic acid or a sugar chain.

3. The method according to Claim 1, wherein the peptide library is composed of random peptide-presenting phage.

4. The method according to Claim 1, wherein the peptide library is composed of random peptide-presenting *Escherichia coli*.

5. The method according to Claim 1, wherein the peptides each have a length of 3 to 15 residues.

6. The method according to Claim 1, wherein the selected recombinant organism or the peptide presented by the recombinant organism is labeled with a labeling substance.


SEQUENCE LISTING

<110> Ajinomoto Co., Inc.

<120> Method For Screening Biomolecule Activity Regulator

<130> B-585AY0P963

<150> JP 11-63110
<151> 1999-03-10

<160> 20

<210> 1
<211> 10
<212> PRT
<213> Artificial Sequence

<220>
<223> Description of Artificial Sequence: phage display peptide K13

<400> 1
Cys Val Pro Leu Val Cys Ile Phe Arg Cys
 1               5                   10

<210> 2
<211> 10
<212> PRT
<213> Artificial Sequence

<220>
<223> Description of Artificial Sequence: phage display peptide J94

<400> 2
Cys Ser Arg Ile Val Cys Leu Leu Trp Cys
 1               5                   10

<210> 3
<211> 7
<212> PRT
<213> Artificial Sequence

<220>

<223> Description of Artificial Sequence: phage display peptide J93

<400> 3
Cys Trp Leu Phe Leu Trp Cys
 1               5

<210> 4
<211> 7
<212> PRT
<213> Artificial Sequence

<220>
<223> Description of Artificial Sequence: phage display peptide N59

<400> 4
Cys Trp Leu Leu Val Phe Cys
 1               5

<210> 5
<211> 6
<212> PRT
<213> Artificial Sequence

<220>
<223> Description of Artificial Sequence: phage display peptide K5

<400> 5
Cys Ile Ala Val Ile Cys
 1               5

<210> 6
<211> 10
<212> PRT
<213> Artificial Sequence

<220>
<223> Description of Artificial Sequence: phage display peptide K25

<400> 6
Cys Arg Pro Val Met Ala Leu Phe Tyr Cys
 1               5                   10

<210> 7

<211> 9
<212> PRT
<213> Artificial Sequence

<220>
<223> Description of Artificial Sequence: phage display peptide N28

<400> 7
Ile Trp Ala Val Leu Trp Ile Trp Asn
 1               5

<210> 8
<211> 9
<212> PRT
<213> Artificial Sequence

<220>
<223> Description of Artificial Sequence: phage display peptide J95

<400> 8
Trp Val Phe Phe Trp Leu Ser Arg Pro
 1               5

<210> 9
<211> 9
<212> PRT
<213> Artificial Sequence

<220>
<223> Description of Artificial Sequence: phage display peptide K1

<400> 9
Ile Trp His Phe Ser Phe Met Trp Ile
 1               5

<210> 10
<211> 10
<212> PRT
<213> Artificial Sequence

<220>
<223> Description of Artificial Sequence: phage display peptide N50

<400> 10
Cys Arg Leu Leu Val Lys Val Phe Trp Cys
1               5                   10

<210> 11
<211> 15
<212> PRT
<213> Artificial Sequence

<220>
<223> Description of Artificial Sequence: phage display peptide N51

<400> 11
Gly Arg Arg Phe Gly Ile Val Cys Thr Cys Leu Lys Tyr Phe Val
1               5                   10                  15

<210> 12
<211> 10
<212> PRT
<213> Artificial Sequence

<220>
<223> Description of Artificial Sequence: phage display peptide N70

<400> 12
Cys Ala Leu Met Ser Cys Leu Phe Trp Cys
1               5                   10

<210> 13
<211> 37
<212> DNA
<213> Artificial Sequence

<220>
<223> Description of Artificial Sequence: primer

<400> 13
catggcagat ctttaagtcg actctagagg cctctgc                                37

<210> 14
<211> 37
<212> DNA
<213> Artificial Sequence

<220>
<223> Description of Artificial Sequence: primer

<400> 14
ggccgcagag gcctctagag tctacttaaa gatctgc 37

<210> 15
<211> 16
<212> DNA
<213> Artificial Sequence

<220>
<223> Description of Artificial Sequence: primer

<400> 15
actcggccga cggggc 16

<210> 16
<211> 18
<212> DNA
<213> Artificial Sequence

<220>
<223> Description of Artificial Sequence: primer

<400> 16
ttcggcccca gcggcccc 18

<210> 17
<211> 36
<212> DNA
<213> Artificial Sequence

<220>
<223> Description of Artificial Sequence: primer

<220>
<221> misc_feature
<222> (18)
<223> n=(NNk)x (N=a or g or c or t, k=g or t, x=4 to 15)

<400> 17

actcggccga cggggctngg ggccgctggg gccgaa                                36

<210> 18
<211> 18
<212> DNA
<213> Artificial Sequence

<220>
<223> Description of Artificial Sequence: primer

<400> 18
tgaattttct gtatgggg                                                    18

<210> 19
<211> 23
<212> PRT
<213> Artificial Sequence

<220>
<223> Description of Artificial Sequence: synthetic peptide 4A18-40

<400> 19
Leu Thr Thr Gly Ser Val Val Ile Val Gly Arg Ile Ile Leu Ser Gly
  1               5                  10                  15
Arg Pro Ala Val Val Pro Asp
             20

<210> 20
<211> 4
<212> PRT
<213> Artificial Sequence

<220>
<223> Description of Artificial Sequence: sequence of spacer

<400> 20
Gly Gly Gly Ser
  1